## Europäisches Patentamt

## European Patent Office

## Office européen des brevets

(11) Veröffentlichungsnummer: **0 088 965**
**B1**

(12) **EUROPÄISCHE PATENTSCHRIFT**

(45) Veröffentlichungstag der Patentschrift:
16.07.86

(51) Int. Cl.⁴: **C 07 C 11/02**, C 07 C 1/20

(21) Anmeldenummer: **83102133.2**

(22) Anmeldetag: **04.03.83**

(54) **Verfahren zur Herstellung von Olefinen aus Methanol und/oder Dimethylether.**

(30) Priorität: **13.03.82 DE 3209223**

(43) Veröffentlichungstag der Anmeldung:
**21.09.83 Patentblatt 83/38**

(45) Bekanntmachung des Hinweises auf die Patenterteilung:
**16.07.86 Patentblatt 86/29**

(84) Benannte Vertragsstaaten:
**BE DE FR GB NL**

(56) Entgegenhaltungen:
**EP - A - 0 016 405**
**EP - A - 0 081 683**
**US - A - 4 058 576**

(73) Patentinhaber: **BASF Aktiengesellschaft,
Carl-Bosch-Strasse 38, D-6700 Ludwigshafen (DE)**

(72) Erfinder: **Lehnert, Rudi, Dr., Budapester Strasse 45,
D-6700 Ludwigshafen (DE)**
Erfinder: **Marosi, Laszlo, Dr., Leuschnerstrasse 32,
D-6700 Ludwigshafen (DE)**
Erfinder: **Leutner, Bernd, Dr., Taunusstrasse 17,
D-6710 Frankenthal (DE)**
Erfinder: **Schlimper, Hans-Ulrich, Dr., Im Erlich 74,
D-6720 Speyer (DE)**
Erfinder: **Schwarzmann, Matthias, Dr.,
Carl-Bosch-Strasse 54, D-6703 Limburgerhof (DE)**
Erfinder: **Stabenow, Joachim, Dr., Am Feldrain 22,
D-6940 Weinheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, Olefine aus Methanol und/oder Dimethylether in Gegenwart von Zeolithkatalysatoren herzustellen.

In neuerer Zeit gewinnen Bemühungen, Methanol zur Herstellung von Olefinen zu verwenden, zunehmendes Interesse. Methanol kann man aus Kohle, durch Vergasung und Konvertierung mit Hilfe bekannter Verfahren ölunabhängig herstellen. Gelingt es, Methanol in technisch befriedigender Weise in niedere Olefine umzuwandeln, so können die heute angewendeten Weiterverarbeitungsverfahren der chemischen Industrie auch für die Verwendung von Kohle als Rohstoff beibehalten werden.

In den vergangenen Jahren sind daher mehrere Verfahren entwickelt worden, die die Umwandlung von Methanol und/oder Dimethylether in Olefine zum Gegenstand haben. Solche Verfahren sind beispielsweise beschrieben in der US-PS 4 049 573 und der DE-OS 2 828 835.

Ein gemeinsames Merkmal dieser bekannten Verfahren besteht darin, dass sie zur Umwandlung von Methanol in Olefine Zeolithkatalysatoren verwenden. Neben Unterschieden in der Art des verwendeten Zeolithkatalysators gibt es auch Unterschiede in der Reaktionsführung. So kann man die Umsetzung von Methanol und/oder Dimethylether mit oder ohne Anwendung eines Verdünnungsmittels, bei vollständigem oder nicht vollständigem Umsatz ausführen. Die Umsetzung kann adiabatisch, isotherm aber auch in einem Wirbelbettreaktor durchgeführt werden. Die übliche Anordnung bei Festbettreaktoren besteht aus mehreren Reaktoren, die zueinander parallel geschaltet sind, wobei mindestens ein Reaktor regeneriert wird. Nicht umgesetzte Ausgangsprodukte, Methanol oder Dimethylether, können in den Prozess zurückgeführt werden. In der US-PS 4 052 479 ist z.B. ein Verfahren zur Herstellung von Olefinen aus Methanol beschrieben, wobei Olefine zurückgeführt werden. In der US-PS 4 058 576 ist ein Zweistufenverfahren zur Umsetzung eines niederen Alkohols in Olefine oder Benzin beschrieben, wobei zwei hintereinandergeschaltete Reaktoren zum Einsatz kommen. Zur besseren Kontrolle der Reaktionswärme wird dabei im ersten Reaktor Methanol in Dimethylether umgewandelt und ein Teil der Reaktionswärme abgeführt.

Die bisherigen Erfahrungen zeigen, dass unter technisch realisierbaren Bedingungen die Prozessführung, insbesondere aber die Herstellung von grösseren Mengen von Ethylen, ausserordentlich schwierig ist. Mit dotierten Katalysatoren kann man nur bei geringen Umsätzen an Methanol und/oder Dimethylether hohe Ethylenausbeuten erzielen. Methanol und Dimethylether müssen daher nach Abtrennung von den Kohlenwasserstoffen in den Prozess zurückgeführt werden. An aktiven und hydrophilen Katalysatoren kann man zwar auch bei 100%igem Umsatz hohe Ausbeuten an Ethylen erhalten, wenn man z.B. 25%ige Methanollösung umsetzt. Die Wärmerückgewinnung und die relativ niedrige Standzeit der Katalysatoren, häufiges Regenerieren, verursachen aber zusätzliche Probleme. In den meisten der bisher bekannten Prozesse beobachtet man auch eine irreversible Alterung des Katalysators, dadurch entstehen hohe Katalysatorkosten.

Es wurde nun gefunden, dass man auch bei hohen Methanolkonzentrationen und vollständigem Umsatz Olefine in verbesserten Ausbeuten durch Umsetzung von Methanol und/oder Dimethylether an Zeolithkatalysatoren in 2 hintereinandergeschalteten Reaktoren bei Drücken von 0,1 bis 10 bar, einer stündlichen Katalysatorbelastung von 0,1 bis 100 g Methanol und/oder Dimethylether/g Katalysator, in zwei Stufen, gegebenenfalls in Gegenwart von Verdünnungsmitteln, erhält, wenn in der ersten Umsetzungsstufe bei Temperaturen von 200 bis 400°C und in der zweiten Umsetzungsstufe bei Temperaturen von 450 bis 600°C gearbeitet wird und als Katalysatoren Alumino- und Borosilikatzeolithe vom Pentasil-Typ oder vom Typ ZSM-34 verwendet werden, wobei die Reaktoren entweder mit verschiedenen Katalysatoren beschickt sind oder die darin befindlichen Katalysatoren in verschiedenen Aktivitätszuständen vorliegen.

Die Umsetzung wird in 2 hintereinandergeschalteten Reaktoren durchgeführt und dabei der zu mindestens 20% aus Olefinen bestehende Produktstrom aus dem ersten Reaktor ohne Abtrennung eines Teiles des Reaktionsproduktes in einen zweiten Reaktor geführt und dort weiter zu einem Produkt mit einem höheren Olefinanteil umgesetzt, wobei die Reaktoren entweder mit verschiedenen Katalysatoren beschickt sind und/ oder die darin befindlichen Katalysatoren in verschiedenen «Aktivitätszuständen» vorliegen und durch die Reaktionsführung mindestens zum Teil verschiedene voneinander entkoppelte Reaktionsschritte der Gesamtumsetzung katalysieren.

Die Umsetzung von Methanol in Dimethylether in einem vorgeschalteten Reaktor wird im Sinne der Erfindung nicht als eine getrennte Stufe angesehen, da der Produktstrom aus dem ersten Reaktor gemäss der vorliegenden Erfindung mindestens 20% Olefine enthalten soll.

Eine besondere Ausführungsform besteht darin, dass man einen Etagenreaktor mit einer in den einzelnen Katalysatorschichten getrennt regelbaren Temperatur verwendet, wobei mindestens eine der Katalysatorschichten mit einem von den anderen Schichten verschiedenen Katalysator beschichtet ist oder in einem anderen «Aktivitätszustand» vorliegt. Man kann aber auch hintereinandergeschaltete Wirbelschichtreaktoren oder einen mehrstufigen Wirbelschichtreaktor verwenden, wobei diese Reaktoren entweder mit verschiedenen Katalysatoren beschickt sind oder die darin befindlichen Katalysatoren sich in unterschiedlichen «Aktivitätszuständen» befinden. Ebenso kann man hintereinandergeschaltete Wanderbettreaktoren oder einen mehrstufigen Wanderbettreaktor verwenden, die mit verschiedenen Katalysatoren beschickt sind oder die darin befindlichen Katalysatoren sich in verschiedenen «Aktivitätszuständen» befinden.

Es war überraschend, dass durch die erfindungsgemässe Reaktionsführung bei 100% Umsatz auch bei Verwendung von 50- bis 100%igen Methanollösungen im Vergleich zu einem einstufigen Verfahren erhöhte Olefine, insbesondere erhöhte Ethylenausbeuten, erhalten werden.

Ein besonderer Vorteil des erfindungsgemässen Verfahrens besteht darin, dass durch Hintereinanderschalten von mehreren Katalysatoren in den einzelnen Reaktionszonen die verschiedenen Reaktionen der Gesamtumsetzung, z.B. Bildung von Ethylen, Homologisierung von Olefinen oder Krackung von Olefinen und Paraffinen entkoppelt werden können und mit Hilfe von verschiedenen Katalysatoren oder durch verschiedene «Aktivitätszustände» desselben Katalysators vorteilhafter in die gewünschte Richtung zu lenken sind als es durch die Verwendung eines einzelnen, auch multifunktionellen, Katalysators in nur einem Reaktor möglich ist. Die Steuerung der Katalyse wird erheblich erleichtert.

Als Katalysatoren werden Alumino- und Borosilikatzeolithe vom Pentasil-Typ oder vom Typ ZSM-34 verwendet. Unter «Aktivitätszustände» des Katalysators im Sinne der Erfindung versteht man z.B. die Aktivität in Abhängigkeit von der Temperatur, dem Kohlegehalt oder der Verdünnung mit amorphen Bindemitteln. Es können auch dotierte oder ionenausgetauschte Formen desselben Zeolithtyps zum Einsatz kommen, oder auch verschiedene Zeolithtypen.

Geeignete Reaktortypen zur Durchführung des erfindungsgemässen Verfahrens sind z.B. der «Etagenreaktor» mit getrennt regelbarer Temperatur in den einzelnen Katalysatorzonen oder mehrere hintereinandergeschaltete Wirbelschicht-, Wanderbett- oder adiabatische oder isotherme Reaktoren. Die Reaktoren können, wie z.B. in Etagenreaktor, als Reaktoren mit getrennten Heizungssystemen oder getrennten Katalysatorzonen ausgestaltet sein. Erfindungswesentlich ist das Vorliegen von verschiedenen Katalysatorzonen mit unterschiedlicher Aktivität. Diese unterschiedliche Aktivität kann durch den Einsatz von verschiedenen Katalysatoren und/oder durch verschiedene «Aktivitätszustände» desselben Katalysators erreicht werden.

Bei dem erfindungsgemässen Verfahren wird in erster Stufe bei Temperaturen von 200 bis 400°C, insbesondere 280 bis 350°C, in zweiter Stufe bei Temperaturen von 450 bis 600°, insbesondere von 530 bis 580°C gearbeitet.

Von bekannten Reaktoranordnungen, in den sich entlang der Katalysatorschicht beim Durchgang der Stoffe auch von sich aus unterschiedliche «Aktivitätszustände» des Katalysators ausbilden können, unterscheidet sich die Anordnung für das erfindungsgemässe Verfahren durch die getrennte regelbare Anordnung von Schichten mit Steuermöglichkeit in denen sich die «Aktivitätszustände» des Katalysators ausbilden bzw. durch eine Anordnung von verschiedenen Katalysatoren in diesen getrennten Schichten.

Eine Auftrennung des Produktstromes wird vorteilhafterweise erst nach dem vollständigen Umsatz von Methanol und/oder Dimethylether vorgenommen. Nach der Auftrennung kann man die Olefine in an sich bekannter Weise weiterverarbeiten, z.B. durch «steam-cracken» eines Teils der Produkte, Homologisierung oder Cracken von Olefinen an zeolithischen oder anderen Katalysatoren oder beispielsweise die Umwandlung von Propylen in Ethylen und Buten (Triolefinprozess) oder nach anderen Weiterverarbeitungsverfahren. Es ist auch möglich, einen Teil der Reaktionsprodukte, z.B. Propylen, Butene oder $C_5^+$-Kohlenwasserstoffe vorzugsweise in eine Zone des Katalysatorsystems zurückzuführen, in der nur noch eine stark verminderte Konzentration an Methanol und/oder Dimethylether vorliegt. Es kann sich auch als vorteilhaft erweisen, in bestimmte Zonen des Katalysatorsystems Wasserdampf oder andere Verdünnungsmittel oder z.B. zwecks Konstanthaltung des Produktspektrums Methanol oder Dimethylether zusätzlich zu dem Produktstrom einzuleiten.

Eine vorteilhafte Ausführungsform besteht z.B. darin, im 1. Reaktor bei unvollständigem Umsatz hohe Ethylenausbeuten zu erzielen und den aus dem ersten Reaktor austretenden Produktstrom in dem weiteren Reaktor oder der weiteren Reaktionszone vollständig umzusetzen. Die Durchführung des erfindungsgemässen Verfahrens wird an Hand der nachstehenden Beispiele erläutert, die die verschiedenen Varianten der Reaktionsführung veranschaulichen. In Beispiel 1 wird gezeigt, dass mit Hilfe des erfindungsgemässen Verfahrens durch Einleiten des Produktstromes aus dem 1. Reaktor in eine weitere Katalysatorzone unter Mehrung des relativen Olfinanteils ein vollständiger Umsatz an Methanol und Dimethylether bei verlängerter Laufzeit zwischen zwei Regenerierungen möglich ist.

Beispiel 1

Der erste Reaktor ist ein Etagenreaktor mit drei Katalysatorzonen. Die Zonen enthalten je 7 g eines Zeoliths vom Pentasiltyp mit einem $SiO_2$/ $Al_2O_3$-Molverhältnis von 40 in Form von 3-mm Pillen. Die Gaseingangstemperatur in die einzelnen Reaktionszonen beträgt einheitlich 330°C. Die Beschickung ist eine 50%ige wässrige Methanollösung, die Belastung 8,1 g $CH_3OH$/g Zeolith/h. Die Umsetzung wird bei 1,14 bar durchgeführt.

Die Zusammensetzung der erhaltenen Reaktionsprodukte ist in Tabelle 1 dargestellt. Man erkennt, dass bereits nach kurzer Laufzeit der Umsatz nachlässt und das Produktgas grössere Mengen Dimethylether aufweist. Reaktor 2 ist ein adiabatischer Reaktor, der mit 30 g Borsilikatzeolithen vom Pentasil-Typ beschickt ist. Das $SiO_2$/ $B_2O_3$-Molverhältnis im Zeolithen beträgt 34. Dieser Katalysator liegt in Form von 3-mm-Strängen vor (Bindemittel $Al_2O_3$, Gewichtsverhältnis Zeolith: Bindemittel = 60:40).

Der aus dem ersten Reaktor austretende Produktstrom wird auf 500°C aufgeheizt und in den 2.

Reaktor eingeleitet. Die Zusammensetzung der erhaltenen Produkte ist in Tabelle 1 dargestellt.

Man erkennt, dass der Umsatz an Methanol und Dimethylether 100% beträgt und der Produktstrom einen erhöhten Olefinanteil aufweist. Die Selektivität zu Ethylen ist grösser als 20 Gew.-%, bezogen auf das eingesetzte $CH_2$.

Beispiel 2

Der erste Reaktor besteht aus einem Etagenreaktor mit 4 Katalysatorzonen. Die Zonen enthalten je 7 g eines Zeolithen vom Pentasiltyp mit einem $SiO_2/Al_2O_3$-Molverhältnis von 40 in Form von 3-mm-Pillen. Die Gaseingangstemperatur in die einzelnen Reaktionszonen beträgt einheitlich 300°C. Die Beschickung ist Rohmethanol (83% Methanol), die Belastung beträgt 10 g $CH_3OH$/g Zeolith/h. Die Umsetzung wird 1,2 bar durchgeführt.

Der Produktstrom wird auf 500°C aufgeheizt und in einen 2. Reaktor geleitet. Der 2. Reaktor ist mit 30 g eines Borsilikatzeolithen vom Pentasiltyp beschickt. Das $SiO_2/B_2O_3$-Molverhältnis im Zeolithen beträgt 34. Dieser Katalysator liegt in Form von 3-mm-Strängen vor.

Aus Tabelle 1 ist die Ethen- und Propanausbeute zu ersehen. Es ist zu erkennen, dass der Anteil an $C_2$-$C_3$-Olefinen beim Durchgang durch den 2. Reaktor steigt und der Anteil an flüssigen Kohlenwasserstoffen abnimmt.

Beispiel 3

Der erste Reaktor ist ein Wirbelreaktor gefüllt mit 75 g eines Katalysators auf der Grundlage eines Alumosilikatzeolithen des Pentasiltyps mit einem $SiO_2/Al_2O_3$-Molverhältnis von 40. Der Katalysator wurde mit $Al_2O_3$ konfektioniert und die Kornfraktion von 0,1 bis 0,5 mm benutzt. Die Betttemperatur betrug 270 bis 300°C. Die Reaktionslösung ist Rohmethanol (83% Methanol), die Belastung bezogen auf den Aluminosilikatzeolithen betrug 3,5 h$^{-1}$. Die Umsetzung wird bei 1,1 bar durchgeführt.

Die Zusammensetzung der erhaltenen Reaktionsprodukte ist in Tabelle 2, Spalte 2 dargestellt. Man erkennt, dass der Umsatz unvollständig ist. Der Produktstrom aus Reaktor 1 wird aufgeheizt auf 500 bis 560°C in einen zweiten Wirbelreaktor eingeleitet.

Der zweite Wirbelreaktor ist mit 60 g eines Borosilikatzeolithen des Pentasiltyps beschickt. Das $SiO_2/B_2O_3$-Molverhältnis beträgt 40. Der Zeolith wurde mit $Al_2O_3$ konfektioniert. Die Kornfraktion lag zwischen 0,1 bis 0,5 mm. Die Zusammensetzung der erhaltenen Produkte ist in Tabelle 2, Spalte 4 und 5 zu ersehen.

In Tabelle 2, Spalte 3 ist das Produktspektrum abgebildet, das der Katalysator im 2. Wirbelbett unter den erfindungsgemässen Bedingungen mit Rohmethanol liefert. Man erkennt, dass der Umsatz von Methanol und Dimethylether 100% beträgt und der Produktstrom einen erhöhten Olefinanteil aufweist. Die Selektivität zu Ethen ist grösser als 20% m/m bezogen auf das eingesetzte $CH_2$.

Durch geeignete Temperaturführung können mit dieser Kombination auch selektiv flüssige Kohlenwasserstoffe hergestellt werden wie in Tabelle 2, Spalte 6 zu erkennen ist.

## Tabelle 1

| Katalysator | Alumosilikatzeolith | Stufe 1: Alumosilikatzeolith Stufe 2: Borosilikatzeolith | Alumosilikatzeolith | Stufe 1: Alumosilikatzeolith Stufe 2: Borosilikatzeolith |
|---|---|---|---|---|
| Methanol: | 50 | 50 | 50 | 83 | 83 |
| Belastung (h$^{-1}$) | 8 | 8 | 8 | 10 | 10 |
| Eingangstemp. (°C) | 330 | 330 | 330/500 | 300 | 300/500 |
| Selektivität (% m/m) bezogen auf eingesetztes $CH_2$ | | | | | |
| Ethen | 23 | 22 | 25 | | 22 |
| Propen | 17 | 18 | 24 | 34 | 38 |
| $\Sigma\ C_4$ | 20 | 16 | 20 | 16 | 15 |
| $\Sigma\ C_5^+$ | 36 | 25 | 16 | 30 | 15 |
| Laufzeit (h) | 3 | 5 | 10 | 5 | 8 |
| Umsatz | 100% | 50–60% | 100% | 100% | 100% |

Tabelle 2

| Katalysator | Alumosilikat-katalysator | Alumosilikat-katalysator | Borosilikat-katalysator | R 1 Alumosilikatzeolith<br>R 2 Borosilikatzeolith | | |
|---|---|---|---|---|---|---|
| Methanol: | 83 | 83 | 83 | 83 | 83 | 83 |
| Belastung (h$^{-1}$): | 3,5 | 3,5 | 3,5 | 3,5 | 3,5 | 3,5 |
| Eingangstemperatur (°C): | 400 | 270–300 erf. Bed. | 500 | 300/500 | 270/560 | 350/460 |
| $CH_4$ | 1,8 | 1,0 | 1,4 | 1,0 | 3,0 | 1,0 |
| Ethen | 16,9 | 24,0 | 9,0 | 21,0 | 24,0 | 7,0 |
| Ethan | 0,5 | 0,2 | 0,2 | – | 1,6 | – |
| Propen | 15,1 | 17,0 | 28,0 | 26,0 | 30,0 | 8,0 |
| Propan | 11,2 | 3,5 | 4,0 | 5,0 | 8,0 | 11,0 |
| $C_4$ | 28,3 | 11,0 | 20,5 | 17,0 | 19,0 | 24,0 |
| $C_5^+$, Gas | 14,3 | 11,0 | 12,0 | 14,0 | 8,0 | 13,0 |
| Öl | 11,5 | 4,0 | 24,5 | 16,0 | 7,0 | 36,0 |
| Umsatz: | 100% | 50–70% | 100% | 100% | 100% | 100% |

**Patentansprüche**

1. Verfahren zur Herstellung von Olefinen durch Umsetzung von Methanol und/oder Dimethylether an Zeolithkatalysatoren in 2 hintereinandergeschalteten Reaktoren bei Drücken von 0,1 bis 10 bar, einer stündlichen Katalysatorbelastung von 0,1 bis 100 g Methanol und/oder Dimethylether/g Katalysator, in zwei Stufen, gegebenenfalls in Gegenwart von Verdünnungsmitteln, dadurch gekennzeichnet, dass in der ersten Umsetzungsstufe bei Temperaturen von 200 bis 400°C und in der zweiten Umsetzungsstufe bei Temperaturen von 450 bis 600°C gearbeitet wird und dass als Katalysatoren Alumino- und Borosilikatzeolithe vom Pentasil-Typ oder vom Typ ZSM-34 verwendet werden, wobei die Reaktoren entweder mit verschiedenen Katalysatoren beschickt sind oder die darin befindlichen Katalysatoren in verschiedenen Aktivitätszuständen vorliegen.

2. Verfahren nach Anspruch 1, dadurch gekennzeichnet, dass man anstelle von 2 Reaktoren einen Etagenreaktor mit getrennt regelbarer Temperatur in den einzelnen Katalysatorzonen verwendet.

3. Verfahren nach Anspruch 1, dadurch gekennzeichnet, dass man hintereinandergeschaltete Wirbelschichtreaktoren oder einen mehrstufigen Wirbelschichtreaktor verwendet.

4. Verfahren nach Anspruch 1, dadurch gekennzeichnet, dass man hintereinandergeschaltete Wanderbettreaktoren oder einen mehrstufigen Wanderbettreaktor verwendet.

**Claims**

1. A process for the preparation of olefins by reacting methanol and/or dimethyl ether over zeolite catalysts in 2 reactors in series at a pressure of from 0.1 to 10 bars and an hourly space velocity of from 0.1 to 100 g of methanol and/or dimethyl ether per g of catalyst, in two stages, in the presence or absence of a diluent, wherein a temperature of from 200 to 400°C is employed in the first reaction stage, and a temperature of from 450 to 600°C is employed in the second reaction stage, and wherein aluminosilicate and borosilicate zeolites of the pentasil or ZSM-34 type are used as catalysts, the reactors being charged with different catalysts or the catalysts present therein being in different states of activity.

2. A process as claimed in claim 1, wherein a tray-type reactor where the temperature in each of the individual catalyst zones can be separately adjusted is used instead of two reactors.

3. A process as claimed in claim 1, wherein fluidized bed reactors in series or a multistage fluidized bed reactor are used.

4. A process as claimed in claim 1, wherein moving bed reactors in series or a multistage moving bed reactor are used.

**Revendications**

1. Procédé pour la préparation d'oléfines par transformation de méthanol et/ou de diméthyléther en présence de catalyseurs zéolitiques dans deux réacteurs montés en série, sous des pressions de 0,1 à 10 bar, avec une charge horaire du catalyseur de 0,1 à 100 g de méthanol et/ou de diméthyléther/g de catalyseur, en deux temps, le cas échéant en présence de diluants, caractérisé en ce qu'on opère, dans le premier temps de la transformation, à des températures de 200 à 400°C et, dans le second temps de la transformation, à des températures de 450 à 600°C et en ce qu'on utilise, comme catalyseurs, des zéolites alumino- et borosilicates du type pentasil ou du type ZSM-34, les réacteurs étant chargés de catalyseurs différents ou les catalyseurs qui s'y trouvent se présentant dans des états d'activité différents.

2. Procédé selon la revendication 1, caractérisé en ce qu'à la place de deux réacteurs, on utilise un réacteur à étages avec température réglable séparément dans les différentes zones de catalyseur.

3. Procédé selon la revendication 1, caractérisé en ce qu'on utilise des réacteurs à couche fluidisée montés en série ou un réacteur à couche fluidisée à plusieurs étages.

4. Procédé selon la revendication 1, caractérisé en ce qu'on utilise des réacteurs à lit mouvant montés en série ou un réacteur à lit mouvant à plusieurs étages.